# EUROPÄISCHE PATENTSCHRIFT

(11) **EP 2 352 210 B1**
(45) Veröffentlichungstag und Bekanntmachung des Hinweises auf die Patenterteilung: **30.03.2016**
(21) Anmeldenummer: 10012895.8
(22) Anmeldetag: 17.01.2003
(51) Int. Cl.: H01S 3/11, A61F 9/008, B23K 26/00

(54) **Femtosekunden-Lasersystem zur präzisen Bearbeitung von Material und Gewebe**
Femtosecond laser system for precise processing of material and tissue
Système à laser femtosecondes pour le traitement précis de matériaux et de tissus

(30) Priorität: 18.01.2002 DE 10202036
(43) Veröffentlichungstag der Anmeldung: 03.08.2011
(62) Teilanmeldung aus: 06018980.0
(73) Patentinhaber: Carl Zeiss Meditec AG, 07745 Jena (DE)
(72) Erfinder: Bergt, Michael, 07745 Jena (DE); Dick, Manfred, 07926 Gefell (DE)
(74) Vertreter: DTS Patent- und Rechtsanwälte Schnekenbühl und Partner mbB

(56) Entgegenhaltungen:
- DE-A1- 19 746 483
- DE-A1- 19 831 674
- DE-A1- 19 907 722
- DE-C1- 19 727 573
- US-A- 4 646 308
- US-A- 5 520 679
- US-A- 5 870 421

## Beschreibung

Die Erfindung betriftt ein Femtosekunden-Lasersystem zur präzisen Bearbeitung von Material und Gewebe, insbesondere ein Lasergerät zur präzisen, mikrometer genauen Bearbeitung von organischem Material, bevorzugt einem Auge.

In einem wertvollen Beitrag zum Stand der Technik wird in der Patentschrift DE 197 46 483 der Anmelderin beschrieben, wie mit Mikrometerpräzision bei der großflächigen Bearbeitung von Materialien mit Lasern mit großem Spotdurchmesser (mm - cm) makroskopische Materialmengen ablatiert, verdampft oder geschmolzen werden (CO₂-Laser Nd:YAG, Excimer ...).

In einem weiteren wertvollen Beitrag zum Stand der Technik wird in der Patentschrift DE 197 27 573 der Anmelderin ein Algorithmus beschrieben, wie ein Laserstrahl abgelenkt werden kann, um eine bestmögliche und präzise Bearbeitung von Material zu gewährleisten.

In der US 5, 656, 186 wird ein Verfahren zum Bearbeiten vom Material bei gleichzeitiger Vermeidung oder Minimierung von schädigenden Nebenwirkungen (Schmelzränder, thermische Schädigung, akustische Schockwellen, Rissbildung) durch Wahl einer speziellen Pulsdauer in Abhängigkeit vom Material beschrieben.

Die materialbearbeitende Wirkung des Lasers ist dabei auf den kleinen Raumbereich des Laserfokus (typischerweise einige µm³) beschränkt, in dem die Lichtintensität hoch genug ist, um die Schwelle des optischen Durchbruchs zu überschreiten. Lokalisiert auf dieses Fokusvolumen wird der Zusammenhalt des Materials zerstört und es entsteht eine Kavitationsblase. Wird der Laserfokus für jeden Laserpuls an eine neue Position gelenkt, können lineare, flächige oder dreidimensionale Schnittmuster generiert werden. Der Abstand benachbarter Kavitationsblasen muss am Ende der Bearbeitung etwa dem Fokusdurchmesser entsprechen, damit das Material entlang der Schnitte leicht mechanisch ablösbar ist.

Die bestehenden Lasergeräte für die Materialbearbeitung mit Femtosekunden-Laserpulsen verwenden regenerative Verstärker, mit denen einzelne Pulse eines Femtosekundenoszillators verstärkt werden. Während der Oszillator selbst nur Pulsenergien im Nanojoule Bereich bereitstellt, können die Pulse mit einem regenerativen Verstärker bis zu einigen Millijoule Pulsenergie verstärkt werden. Die Repetitonsraten sind durch die Verwendung von Pockelszellen zur Ein- und Auskopplung in den Verstärker auf einige 10 kHz beschränkt. Während diese Laserquellen für Anwendungen mit hohen Abtragsraten pro Laserpuls geeignet sind, sind sie nicht optimal für die oben beschriebene Anwendung für Präzisionsschnitte.

In K. König et al., Optics Letters Vol. 26, No. 11 (2001*)* wurde beschrieben, wie auch mit Nanojoule-Pulsen aus einem Femtosekunden-Oszillator Schnitte in Gewebe ausgeführt werden können. Da dabei aber ein einzelner Laserpuls nicht zur Ausbildung einer Kavitationsblase führt, sondern mehrere, an die gleiche Stelle plazierte Pulse nötig sind, um eine Schnittwirkung zu erzielen, eignet sich dieses Verfahren nur für sehr feine Schnittfiguren im Mikrometermaßstab. Für den industriellen bzw. medizinischen Einsatz ist diese Laserquelle nicht geeignet.

In der DE 199 07 722 A1 wird ein Lasersystem zur Erzeugung ultrakurzer Lichtimpulse beschrieben mit einem Laserresonator, der zumindest ein aktives Festkörper-Oszillatorelement enthält, vorzugsweise eine dünne Scheibe eines laseraktiven Kristalls mit hoher Verstärkungsbandbreite, und zumindest je eine Einrichtung zur Phasenverkopplung der Lasermoden und zur Dispensionskompensation aufweist.

Aufgabe der vorliegenden Erfindung ist es daher, eine Vorrichtung zur präzisen Bearbeitung von Material bereitzustellen, mit der diese Nachteile des Standes der Technik überwunden werden.

Diese Aufgabe wird durch die Vorrichtung nach dem unabhängigen Ansprüch gelöst. Weitere vorteilhafte Ausgestaltungen sind in den abhängigen Ansprüchen angegeben.

Insbesondere wird die Aufgabe gelöst durch eine Vorrichtung zur präzisen Bearbeitung von Material, insbesondere organischem Material, umfassend ein gepulstes Lasersystem mit einer Strahlquelle, wobei ein cavity-dumped fs-Oszillator als Strahlquelle vorgesehen ist.

Die Aufgabe wird des Weiteren gelöst durch eine Vorrichtung zur präzisen Bearbeitung von Material, insbesondere organischem Material, umfassend ein gepulstes Lasersystem mit einem cavity-dumped fs-Oszillator als Strahlquelle, bei dem durch eine Strahleinrichtungen mit mindestens einem Mittel zur Strahlablenkung ein Arbeitsstrahl der Strahlquelle auf das Material applizierbar ist, wobei die Pulsaussendung mit der Strahlablenkung korrelliert und wobei das Mittel zur Strahlablenkung Mittel zur Freigabe von Laserpulsen umfasst. Unter Freigabe wird dabei verstanden, dass der Laser für einen Laserimpuls freigegeben wird und der Laserimpuls ausgelöst wird, sobald der Laser entspechend seiner maximalen Repetitionsrate erneut einen Laserimpuls abgeben kann. Unter Korrelation der Pulsaussendung mit der Strahlablenkung wird insbesondere verstanden, dass die Pulsaussendung erfolgen kann, wenn der Strahl auf einen bestimmten Punkt gelenkt wurde, die Pulsaussendung also in Abhängigkeit der Strahlablenkung angesteuert wird.

Die Aufgabe wird ebenfalls gelöst durch eine Vorrichtung zur präzisen Bearbeitung von Material, insbesondere organischem Material, umfassend ein gepulstes Lasersystem mit einem cavity-dumped fs-Oszillator als Strahlquelle, wobei die Energie der Strahlung etwa 100 nJ bis 10 µJ, vorzugsweise 500 nJ bis 5 µJ, beträgt. Die Repetitionsrate der Strahlung beträgt dabei vorzugsweise 50 khz bis 1 Mhz, besonders bevorzugt 100 khz bis 500 khz. Der Fokusdurchmesser der Strahlung beträgt dabei vorzugsweise etwa 500 nm bis 10 µm, besonders bevorzugt 3µm bis 5µm. Die Pulsdauer der Strahlung beträgt vorzugsweise etwa 100 fs bis 1 ps, besonders bevorzugt 200 fs bis 300 fs.

Die Mittel zur Strahlformung und/oder Strahlablenkung bzw. allgemeiner formuliert die Strahlformungs- und - ablenkungssysteme können diffraktive oder refraktive Mikrooptiken oder adaptive Optiken oder klassische optische Systeme umfassen. Mit difraktiven oder refraktiven Elementen kann man dabei mehrere klassische bzw. konventionelle optische Elemente ersetzen. Bei Verwendung eines derartigen relativ aufwändigen Strahlformungs- und -ablenkungssystems können vergleichsweise einfache Laser eingesetzt werden.

Die genannte Vorrichtung zur präzisen Bearbeitung von Material wird vorzugsweise eingesetzt für die ophtalmologische Augenbehandlung, insbesondere zur Korrektur der Fehlsichtigkeit eines Auges. Die Vorrichtung kann zum Schneiden eines Flaps oder Lentikels in der Cornea zur Korrektur der Fehlsichtigkeit verwendet werden. Neben einem schneiden des Lentikels können mit der erfindungsgemäßen Vorrichtung refraktive Strukturen in der Cornea, beispielsweise in Form flächenmäßig nebeneinander gesetzter Spots oder einer Punktwolke, erzeugt werden.

Ebenso können unmittelbar Laserschüsse zur Erzeugung refraktiver Strukturen gesetzt werden. Beispielsweise können in der Augenlinse kleine Bläschen durch verdampfen von Material bzw. Flüssigkeit erzeugt werden. Dazu sind sehr viele Laserschüsse mit vergleichsweise niedriger Energie erforderlich, wie sie mit der erfindungsgemäßen Vorrichtung bereit gestellt werden können.

Ebenso ist es möglich, mit der erfindungsgemäßen Vorrichtung geziehlte Schnitte in das Gewebe, beispielsweise der Augenlinse, einzubringen und damit die Krümmbarkeit und Elastizität der Augenlinse zu verbessern, da sich die benachbarten Gewebeteile nun leichter gegeneinander verschieben lassen. Die Vorrichtung zur präzisen Bearbeitung von Material, insbesondere organischem Material, wird in dieser Ausgestaltung der Erfindung als Vorrichtung zur Behandlung der Presbyopie eingesetzt. Die Strahlformung erfolgt entweder konventionell oder mit diffraktiven bzw. refraktiven Mikrooptiken oder adaptiven Optiken. Die Strahlablenkung erfolgt vorzugsweise über Scansysteme.

Eine ideale Quelle stellen direkt diodengepumpte Femtosekundenoszillatoren (evtl. mit SESAM (semiconductor saturable absorber mirror) zum Start und Stabilisieren des Modelocking und dispersiven Spiegeln (chirped mirrors) zur Dispersionskompensation im Resonator dar. Durch "cavity dumping" können statt der normalerweise mit mehreren 10 MHz emittierten Nanojoule-Pulse dann Mikrojoule Pulsenergien bei verringerter Repetitionsrate erzeugt werden. Dabei wird, anstatt bei jedem Umlauf eines Laserpulses im Resonator einen geringen Teil der intracavity-Pulsenergie (typisch ca. ein Prozent) passiv auszukoppeln, der Laserpuls für mehrere Umläufe vollständig im Resonator belassen und dann einmalig ein größerer Anteil der Laserleistung aktiv aus dem Resonator ausgekoppelt. Die verbleibende Laserpulsenergie wird dann in mehreren Umläufen im Resonator wieder auf einen Sättigungswert verstärkt bis ein weiterer Puls ausgekoppelt wird. Bei gleicher mittlerer Laserleistung von einigen 100 mW werden so Laserpulse bis hin zum Mikrojoulebereich bei Repetitionsraten von 0 - einigen MHz erzeugt. Zur aktiven Auskopplung der Laserpulse können elektrooptische oder akustooptische Deflektoren mit schnellen Schaltzeiten verwendet werden. Besonders bevorzugt werden Pulsdauern von unter 10ps, bevorzugt deutlich kürzer als 1ps bevorzugt 100-200 fs erzeugt.

Das bevorzugt mit Mikrometergenauigkeit zu bearbeitende Material kann Material mit Strukturen im Mikrometerbereich, Gitter, Kontaktlinsen, Kunststoffe, Intraokkularlinsen (IOL), Halbleiterwafer, mikrooptische Elemente etc. umfassen. Besonders bevorzugt ist organisches Material, wie beispielsweise Gewebe, besonders bevorzugt das menschliche Auge.

Das gepulste Lasersystem ist eine Anordnung einer Laserstrahlquelle zur Erzeugung von fs-Pulsen und entsprechenden optischen Vorrichtungen, insbesondere Spiegel, Linsen, etc.

Durch diese Laservorrichtung wird ein System bereitgestellt, dessen Pulsenergien bei gleichzeitig hoher Repetitionsrate mikroskopische und makroskopische Schnittfiguren bevorzugt mit Mikrometergenauigkeit ermöglichen. Werden - wie im Stand der Technik - Oszillatoren in regenerativen Verstärker nachverstärkt um Mikrojoule-Pulsenergien zu erzeugen, begrenzen die zur Ein- und Auskopplung in den Verstärker verwendeten Pockelszellen die Repetitionsrate des Laserverstärkersystems. Diese Nachteile existieren bei dem erfindungsgemäßen Laser nicht. Die erfindungsgemäße Vorrichtung ist in der Lage, Mikrojoule-Pulsenergien bei einstellbaren Repetitionsraten von Null bis hin zu einigen MHz zu generieren. Besonders bevorzugt werden Pulsenergien von wenigen Mikrojoule und Repititionsraten von einigen 100 kHz verwendet. Die Oszillatoren arbeiten dann mit mittleren Leistungen von einigen 100 mW. Besonders bevorzugt wird als Strahlquelle ein direkt diodengepumpter Oszillator verwendet. Dieser ist besonders einfach und zuverlässig.

In einer Ausgestaltung der erfindungsgemäßen Vorrichtung ist vorgesehen, dass das Mittel zur Strahlablenkung im Scan-Modus betrieben werden. Der Arbeitsstrahl der Strahlquelle kann dabei auf in einer Dimension periodisch wiederkehrenden Bahnen abgelenkt werden, sodass beispielsweise kreisförmige Bahnen unterschiedlicher Durchmesser oder spiralförmige Bahnen erzeugt werden können. Die Bahnen des Arbeitsstrahles können durch eine rotierende oder in anderer Weise auf einer Bahn gehaltenen Vorrichtung, beispielsweise durch einen Spiegel, eine Linse, ein Gitter oder dergleichen, erzeugt werden. Die Mittel zur Strahlablenkung können Scanner, z.B. mechanische Scanner, umfassen, die auf vorgegebenen Bahnen bewegbar gelagert sind. Die vorliegende Erfindung nutzt Ablenksysteme, die den Laser auf den natürlichen Bahnen des Ablenksystems ablenkt, also z.B. auf Kreisbahnen bei rotierenden Ablenksystemen. Anstatt einzelne Positionen anzufahren und dort einen Laserimpuls auszulösen, sobald die vorgegebene Position erreicht ist und das Ablenksystem wieder ruht, wird die Bahn des Ablenksystems ohne Stops durchlaufen und die Pulse werden durch eine vorgewählte, über die Bahngeschwindigkeit der Fokusbewegung vorgegebene Repetitionsrate beginnend zu einem definierten Zeitpunkt abgegeben. Sobald also die Fokusposition einen bestimmten Punkt erreicht hat, wird der Laser freigegeben bzw. getriggert, also zur Abgabe eines Laserimpulses angesteuert. Unter Freigabe wird dabei verstanden, dass der Laser für einen Laserimpuls freigegeben wird und dieser Laserimpuls ausgelöst wird, sobald der Laser entspechend seiner maximalen Repetitionsrate erneut einen Laserimpuls abgeben kann. Dies führt zu einer Spur von Wirkvolumina, mithin durch den Laserfokus während der kurzen Pulsdauer modifizierte Stellen im Material, entlang einer im wesentlichen vordefinierten Bahn, die insbesondere dadurch ausgezeichnet ist, dass benachbarte Wirkvolumina in gleichbleibendem, vordefiniertem Abstand, beispielsweise in der Größenordnung des Fokusdurchmessers, plaziert werden. Durch leichte Modifikation der natürlichen Bahn des Ablenksystems, z.B. durch eine leichte Reduktion des Kreisbahnradius, beispielsweise um den Betrag des Abstandes benachbarter Wirkvolumina, können weitere Spuren geschrieben werden, die sich zu einer Schnittfläche ergänzen. Beispielsweise können hier konzentrische Bahnen oder spiralförmige Bahnen oder dergleichen erzeugt werden. Bei Verwendung eines Ablenkspiegels kann dies beispielsweise durch eine Veränderung der Neigung bei gleichbleibender Rotation des Spiegels geschehen. Ziel ist es, die gewünschte Schnittfläche mit einem gleichmäßigen Raster von Wirkvolumina bzw. Laserfoki zu überdecken. Die natürlichen Bahnen des Ablenksystems können sehr schnell mit definiertem zeitlichem Ablauf durchfahren werden. Die Anpassung der zeitlichen Abfolge der Laserpulse führt dann zur gewünschten Überdeckung der Schnittfläche mit Laserschüssen.

Durch dieses Vorgehen geforderte Eigenschaften der Strahlquelle, nämlich die Triggerbarkeit zu den durch das Ablenksystem vorgegebenen Zeiten, weisen cavity-dumped fs-Oszillatoren auf. Die einzelnen Pulse der Strahlquelle werden also dann ausgelöst, wenn das Ablenksystem die vorgegebene Position erreicht hat. Vorteilhaft sind dabei Bahnbewegungen, in denen Pulse in gleichen zeitlichen Abständen ausgelöst werden können. Bei den hier geforderten kurzen Pulslängen, hohen maximalen Repitionsraten und moderaten Pulsenergien stellen cavity-dumped fs-Oszillatoren derzeit die idealen Strahlungsquellen für das erfindungsgemäße System dar.

Bei einem weiteren Ausführungsbeispiel der vorliegenden Erfindung sind weiterhin Strahleinrichtungen zur Strahlformung und/oder Strahlführung und/oder Strahlablenkung und/oder Strahlfokusierung vorgesehen. Durch diese Strahleinrichtungen kann der Strahl genau so auf das zu bearbeitende Material gelenkt und geleitet werden, wie es die geplante Anwendung erfordert. Die hier auf einen Fokusdurchmesser in der Größenordnung von 1 µm fokussierten ultrakurzen Laserpulse können in einem kleinen, präzise definierten Fokusvolumen den Materialzusammenhalt lösen oder strukturelle Veränderungen im Material hervorrufen ohne benachbarte Gebiete im Material thermisch, akustisch oder mechanisch zu belasten. Für makroskopische Schnitte und Strukturen im Zentimetermaßstab wird der Laserfokus dreidimensional durch das zu bearbeitende Material gescannt. Der Anwendungsfall bestimmt, wie Strahlquelle, Strahlführung und -formung, Scanner, Scanalgorithmus und Fokussieroptik aufeinander abgestimmt werden, um eine hohe Bearbeitungsgeschwindigkeit bei gleichzeitig hoher Präzision zu erreichen.

Die Strahlformung geschieht dabei bevorzugt mittels eines Teleskops (bevorzugt Galilei-Teleskop mit Sammel- und Streulinse), das den Strahldruchmesser so aufweitet, dass der Laser auf einen entsprechend kleinen Fokus fokussiert werden kann. Bevorzugt wird ein Linsensystem verwendet, das die Abbildungsfehler des Teleskops weitgehend minimiert.

Die Strahlführung erfolgt bevorzugt durch Spiegel oder Spiegelpaare, mit denen der Strahl in die einzelnen Subkomponenten justiert werden kann.

Die Strahlablenkung können konventionelle Scanner bzw. mechanische Laserstrahl-Ablenksysteme wie Galvanometerspiegel im Close-Loop-Betrieb, etc. sein. Bevorzugt jedoch sind mechanische Scanner, die vorgegebene Bahnen (z.B. Kreisbahnen) abfahren und durch Triggerung der Strahlquelle an den vorgesehenen Positionen dadurch Laserpulse ausgelöst werden. So kann auf einem großen Bereich der Schnittfläche mit voller Repetitionsrate bei relativ langsamen Scannerbewegungen gearbeitet werden. Soll in noch kleineren Bereichen der Schnittfläche gearbeitet werden (z.B. wenn der Kreisbahnradius zu klein wird) kann bevorzugt zusätzlich die Repetitionsrate des Lasers reduziert werden. In einem kleinen Bereich der Schnittfläche (wenn z.B. der Kreisbahnradius sehr klein wird), wird dann die Repetitionsrate bevorzugt an die dann kleiner werdende maximale Bahngeschwindigkeit, die durch die begrenzte Winkelgeschwindigkeit des Ablenkmechanismus bedingt ist, angepasst und verringert. Bei einer Ablenkeinheit, die auf der Rotation eines optischen Ablenkelements beruht, kann es technologisch einfacher sein, mit konstanter Drehzahl und somit konstanter Winkelgeschwindigkeit der Bahnbewegung des Laserfokus zu arbeiten. Dann kann die Repetitionsrate des Lasers bei jeder kleiner oder größer werdenden Kreisbahn an die neue Bahngeschwindigkeit, die sich aufgrund des veränderten Bahnradius ergibt, angepasst werden. Dabei ergibt sich für die Bearbeitung einer Kreisfläche eine um nur den Faktor 2 erhöhte Bearbeitungsdauer, als wenn alle Spots mit maximaler Repetitionsrate gesetzt würden. Durch Anpassung der Drehzahl der Ablenkeinheit in wenigen Stufen während der Bearbeitung, kann die Bearbeitungsdauer noch weiter verringert werden.

Die Strahlfokussierungseinrichtung dient dazu, im Fokus des Strahls auf oder innerhalb des Materials den Zusammenhalt des Materials aufzuheben (Photodisruption). Im Allgemeinen geht das mit einer lokalen Verdampfung des Materials einher. Bevorzugt wird der Laser hierfür auf einen Durchmesser im Mikrometerbereich fokusiert. Dies liegt nahe am Beugungslimit von Licht im sichtbaren bzw. nahen Infrarotbereich. Die Fokussieroptik weist daher bevorzugt eine hohe numerische Appertur und damit eine kurze Brennweite und eine große optische Öffnung (aufgeweiteter Laserstrahl Durchmesser) aus. Bevorzugt wird der von der Laserquelle ausgehende Strahl vor der Fokussierung auf das Material bzw. Gewebe im Durchmesser aufgeweitet. Die Systeme zur Strahlführung, -ablenkung und - fokussierung sind daher bevorzugt für einen großen Stahldurchmesser ausgelegt.

Laserquelle, Stahlablenkung (Scanner) und Fokussieroptik sind so aufeinander abgestimmt, dass präzise und schnelle Schnittführung im Wege der Fotodisruption ermöglicht wird. Dabei werden Laserspots mit einem Fokusdurchmesser von einigen 100 nm bis einigen µm mit einem Spotabstand in der Größenordnung des Spotdurchmessers im Material platziert.

In einer besonders bevorzugten Ausführungsform sind die Strahleinrichtungen, insbesondere die Ablenkeinrichtungen, programmierbar. Durch die Abstimmbarkeit der einzelnen Strahleinrichtungen aufeinander und die Steuerung durch entsprechende Programme kann das System der Strahleinrichtungen zusammen mit dem gepulsten Lasersystem genau auf das Material und die Schnittanforderung eingestellt werden, für die es eingesetzt werden soll. So kann in Abhängigkeit der Transparenz und Brechkraft des zu bearbeitenden Materials sowie der Anforderung an Schnittgeometrie und Operationsdauer das Set an Parametern durch das Programm vorgewählt und abgestimmt werden.

In einer weiteren bevorzugten Ausführungsform der vorliegenden Erfindung sind weiterhin Haltevorrichtungen zur Positionierung und/oder Fixierung des zu bearbeitenden Materials vorgesehen. Durch diese Haltevorrichtungen wird sichergestellt, dass die mikrometergenauen Strukturen, die durch den erfindungsgemäßen Laser hergestellt werden können, nicht durch unkontrollierbare Bewegungen des zu bearbeitenden Materials, insbesondere Auges, beeinträchtigt werden.

Eine solche Fixier- und Positioniervorrichtung kann eine einfache Klemmvorrichtung für ein Werkstück sein, das bevorzugt mit Mehrachsen-Justagemöglichkeiten zur Bewegung und Verkippung des Werkstücks zur optimalen Justage ausgestattet ist. Fixiereinrichtungen für die medizinische Anwendungen an Organen wie zum Beispiel dem Auge müssen außerdem den jeweiligen biologischen Gegebenheiten angepasst sein. Die Fixierung des menschlichen Auges kann zum Beispiel mit Hilfe eines Kontaktglases und eines Vakuum-Saugringes erfolgen.

Eine Positionierungsvorrichtung kann bevorzugt ein Kontaktglas sein. Dieses Kontaktglas kann entweder eben sein, oder bevorzugt der Krümmung des zu bearbeitenden Materials, insbesondere der Hornhaut, im Wesentlichen angepaßt sein.

Als Fixiervorrichtung wird bevorzugt ein Saugring verwendet. Der Saugring ist bevorzugt fest mit dem Austrittsfenster des Lasergeräts verbunden, was zu einer definierten Lage der Hornhaut relativ zum Laserfokus führt.

Bei einer weiteren Vorrichtung der vorliegenden Erfindung werden durch den cavity-dumped fs-Oszillator Laserpulse von 100 nJ bis 100 µJ, bevorzugt 1 µJ Pulsenergie bereitgestellt. Mit dieser Pulsenergie kann eine Photodisruption in dem angesteuerten Bereich des Materials im Mikrometerbereich ausgelöst und eine Kavitationsblase erzeugt werden, insbesondere wenn die Pulsdauer unter 300 fs liegt. Ein daneben gesetzter Schuß wird benachbart zu dieser Kavitationsblase eine weitere Kavitationsblase erzeugen. Durch diese Blasen wird dann das Materialgefüge dauerhaft oder für einen bis mindestens zum Ende der Bearbeitung dauernden Zeitraum aufgehoben.

Besonders bevorzugt werden durch den cavity-dumped fs-Oszillator Laserpulse mit Repetitionsraten von 10 kHz bis 10 MHz bereitsgestellt. Die Repetitionsrate wird bevorzugt auf die Geschwindigkeit eines Ablenksystems (z.B. maximale Drehzahl der Ablenkoptik) abgestellt. Für die Anwendung am Auge wird bevorzugt eine 300 kHz Repetitionsrate bei ca. 50Hz Drehzahl verwendet, was eine Bearbeitungszeit von unter einer Minute ergibt.

Mit diesen Repetitionsraten kann in Abstimmung mit der vorgegebenen Pulsenergie und der Ablenkeinrichtungen die Laserwirkung für die Photodisruption präzise lokalisiert werden. Hierdurch wird in einem scharf begrenzten Fokusvolumen das Materialgefüge zerstört, in dicht benachbarten Bereichen (von weniger als ein Mikrometer entfernt) findet im Allgemeinen keine Veränderung des Materials statt. Daraus ergibt sich eine hohe Bearbeitungspräzision (Mikrometergenauigkeit) bei Schonung benachbarter Materialregionen. Thermische und mechanische Beanspruchung der nicht bearbeiteten Regionen sind deutlich geringer als bei anderen Bearbeitungsmethoden.

Bei einer weiteren bevorzugten Ausführungsform der erfindungsgemäßen Vorrichtung ist durch die Strahleinrichtung, insbesondere die Ablenkeinrichtung, ein Arbeitsstrahl der Strahlquelle in geometrisch vorbestimmbarer Form in zeitlich vorbestimmbarem Verlauf auf das Material applizierbar. Durch das Zusammenspiel der einzelnen Komponenten ist so möglich, Schnitte und Strukturierungen zu erzeugen. Zur Erzeugung eines Spots, in dem das Materialgefüge aufgelöst wurde, genügt im Allgemeinen ein Laserpuls mit definiertem Pulsparametern (Pulsenergie, Pulsdauer, Fokus). Für Schnitte und Strukturierung ist eine Vielzahl solcher Spots dicht nebeneinander zu plazieren. Der Abstand benachbarter Spots sollte am Ende der Prozedur in der Größenordnung des Spotdurchmessers liegen. Dafür kann der Laserfokus scannend über bzw. durch das Material bewegt werden. Der Laserfokus folgt in idealer Weise 3-dimensional mit Mikrometergenauigkeit einer vorgegebenen geometrischen Bahn. So ist es beispielsweise möglich, einen Schnitt in dem zu bearbeitenden Material dadurch zu erzeugen, dass eine beliebige Fläche, zum Beispiel eine Rechteckfläche benachbarter Mikrometerspots in dem Gewebe nacheinander scannend angesteuert wird. Dadurch wird genau in dieser Ebene der Materialzusammenhalt aufgelöst und dadurch ein "Schnitt" im Gewebe erzeugt. Genauso ist es möglich, den Laserfokus durch Kreisbewegungen des Scanners in einer Kreisbahn auf das zu bearbeitende Material zu applizieren. Durch eine sich anschließende helixförmige Führung des Bearbeitungsstrahls kann so beispielsweise eine Zylinderfläche aus dem Material herausgeschnitten werden. Da die Photodisruption bevorzugt in einem sehr engen Bereich stattfindet, kann der Laserstrahl auch im Gewebe wirken, ohne dass das vom Laserstrahl außerhalb des Fokus durchdrungene Material beschädigt wird. Auf diese Weise sind beliebige geometrische Bahnen und damit Formen durch Photodisruption in dem Material herausschneidbar.

Beim bevorzugten Ausführungsbeispiel der vorliegenden Erfindung ist eine Vorrichtung vorgesehen, bei der der gepulste Arbeitsstrahl durch die Strahlablenkungseinrichtung auf das Material applizierbar ist und währenddessen die Repetitionsrate der Pulse des Arbeitsstrahles modifizierbar ist. Durch das Vorsehen einer Einrichtung zur Modifizierung der Repetitionsrate bei gleichzeitiger Strahlführung des Arbeitsstrahles über das zu bearbeitende Material kann auf diese Weise elegant ein gleichmäßiges Spotmuster auf dem zu behandelnden Material erzeugt werden, auch wenn der Strahl unter verschiedenen Winkeln bzw. verschieden schnell durch die Ablenkeinrichtung auf das zu bearbeitende Material gerichtet wird. Ein besonders augenfälliger Vorteil wird beispielsweise dann erreicht, wenn die Ablenkeinrichtung den Strahl in Kreisbahnen auf das zu bearbeitende Material lenkt und diese Kreisbahnen mit einer speziellen Umlauffrequenz der Ablenkeinrichtung, insbesondere beispielsweise der Ablenkspiegel, erzeugt wird. Wird bei einer Umlauffrequenz von beispielsweise 50Hz der Laserstrahl auf einer Kreisbahn von 1cm Durchmesser bei einer Repetitionsrate von 300kHz geführt, dann werden auf jeder Kreisbahn pro Umlauf gleichmäßig verteilt 60000 Spots gesetzt. Wenn der Strahl dann auf einem Kreis von nur 0,5cm Durchmesser mit derselben Frequenz der Ablenkeinrichtung geführt wird, kann durch Erniedrigung der Repetitionsrate des gepulsten Arbeitsstrahles der gleiche Abstand der einzelnen Spots voneinander auf dem zu bearbeitenden Material erzeugt werden, wie bei der Strahlführung auf der größeren Kreisbahn. Durch eine entsprechende Modifikation der Repetitionsrate in Abhängigkeit der durch die Ablenkeinrichtung abgefahrenen Geometrie lassen sich so beliebige geometrische Muster mit einem im Wesentlichen gleich bleibenden Spotabstand auf dem zu bearbeitenden Material erzeugen. Beispielsweise können Spiralen abgefahren werden, bei denen von außen nach innen bei gleichbleibender Umlauffrequenz der Ablenkeinrichtung die Repetitionsrate immer weiter abnimmt. Daneben sind auch beliebige andere geometrische Formen denkbar. Ist eine konstante Beabstandung der einzelnen Spots auf dem Material gerade nicht beabsichtigt, sondern soll vielmehr in einem speziellen Bereich eine höhere Spotdichte und in einem weiteren Bereich eine niedrigere Spotdichte erreicht werden, so kann dies ebenfalls durch Kombination der gewählten Parameter für die Repetitionsrate des Arbeitsstrahles und die Frequenz bzw. den örtlichen Verlauf der Ablenkeinrichtung erzeugt werden. So ist es bevorzugt auch möglich, graduell unterschiedliche Bereiche mit verschiedener Fokusdichte zu erzeugen. Beispielsweise kann bei einem Kreis das Zentrum einen sehr niedrigen Fokusabstand aufweisen während der Fokusabstand zum Rand hin immer größer wird.

Die Aufgabe wird auch durch ein Verfahren zur Applikation von fs-Pulsen einer cavity-dumped fs-Oszillator Strahlquelle auf ein Material, insbesondere ein organisches Material, gelöst, bei der im Fokus des Laserstrahls das Material mit Photodisruption bearbeitet bzw. dessen Zusammenhalt aufgelöst wird.

Beim besonders bevorzugten Verfahren wird der gepulste Laserstrahl mittels einer Ablenkeinrichtung auf das zu bearbeitende Material gelenkt und in Abhängigkeit des hierdurch auf dem Material erzeugten Spotmusters die Repetitionsrate der Pulse des Laserstrahls modifiziert. Auf diese Weise kann jedes beliebige Spotmuster und insbesondere jede beliebige Beabstandung der einzelnen Spots voneinandern in der gewünschten Geometrie auf dem zu bearbeitenden Material erzeugt werden. Besonders bevorzugt werden die Spotmuster so auf dem zu bearbeitenden Material verteilt, dass die Kavitationsblase jedes einzelnen Spots, die durch Photodisruption entsteht, genau benachbart zu der Kavitationsblase des nächsten Spots gesetzt werden. Auf diese Weise entsteht dann ein gewünschtes Schnittmuster direkt benachbarter Kavitationsblasen. Für spezielle Anwendungsfälle kann es auch gewünscht sein, die Spots noch enger zu setzen. Dies ist beispielsweise dann empfehlenswert, wenn das zu bearbeitende Material sich nach einer gewissen Zeit wieder erneuert und die Ablösung des Materials für eine spezielle Zeit sichergestellt werden soll, bevor beispielsweise der Bohrkern oder ein sonst herausgeschnittenes Stück des zu bearbeitenden Materials entfernt werden kann. Ebenso ist es denkbar, dass die Spots zuerst mit einer größeren Beabstandung gesetzt werden, um in einem folgenden Schritt die Lücken zwischen den Spots zu füllen und dadurch ein gewünschtes Muster von Kavitationsblasen zu bilden.

Die erfindungsgemäße Vorrichtung kann verwendet werden zur refraktiven Chirurgie durch Bearbeitung der Cornea oder Linse des Auges.

Im Folgenden sollen weitere vorteilhafte Ausgestaltungen der Erfindung an Hand der Zeichnung erläutert werden. Hierbei zeigt
- Fig. 1: zeigt eine schematische Darstellung eines Ausführungsbeispiels eines erfindungsgemäßen Lasers
- Fig. 2: zeigt ein weiteres Ausführungsbeispiel eines erfindungsgemäßen Lasers mit Operationsmikroskop und zu bearbeitendem Auge;
- Fig. 3: zeigt eine schematische Darstellung von einigen Beispielen möglicher Schnittmustern, die mit dem erfindungsgemäßen Lasersystem ausgeführt werden können;
- Fig. 4: zeigt schematisch eine Detailansicht einer Folge von Laserspots auf Kreislinien und
- Fig. 5: zeigt den zeitlichen Verlauf von Folgen von Laserpulsen im und außerhalb des Laserresonators.

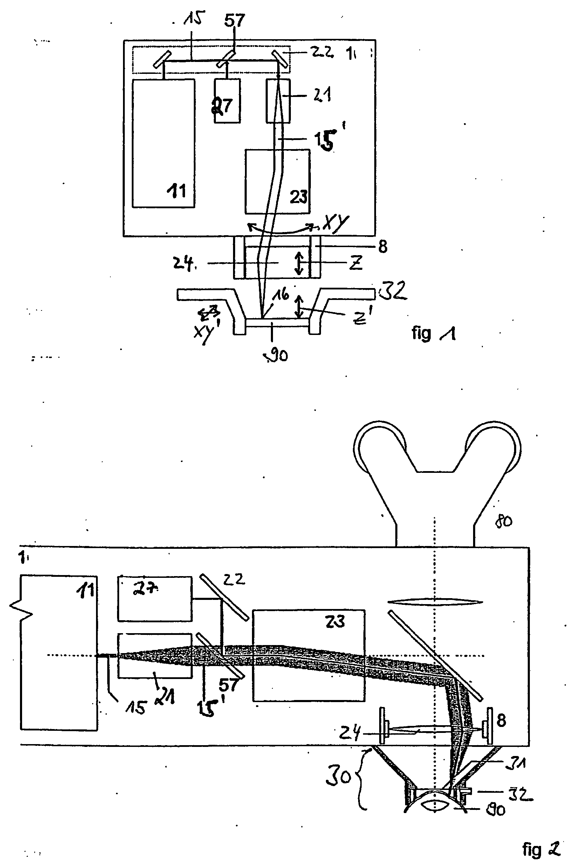

In Figur 1 ist eine schematische Darstellung der einzelnen Komponenten eines Ausführungsbeispiels eines erfindungsgemäßen Lasersystems dargestellt. Die Bearbeitungsvorrichtung 1 umfaßt als Strahlquelle 11 einen cavity-dumped fs-Oszillator. Der Laserstrahl 15 wird über Spiegel und einen Strahlteiler 57 auf eine Strahlaufweitungsoptik 21 ausgekoppelt. Der aufgeweitete Laserstrahl 15' wird dann über eine Strahlablenkungseinrichtung wie beispielsweise einen Scanner in XY-Richtung auf eine Strahlfokussierungseinrichtung 24 gelenkt. Diese ist in der Z-Achse verschiebbar und erlaubt so die Verschiebung des Fokuspunktes durch Verschiebung der Strahlfokussierungseinrichtung entlang des Pfeiles Z. Alternativ kann ein fokussierendes optisches System mit veränderlicher Brennweite verwendet werden, um die Fokusposition in Z-Richtung kontrolliert zu verschieben. Der fokussierte Laserspot 16 wird so auf das zu bearbeitende Material 90 gelenkt, das durch eine Fixierungsvorrichtung 32 in seiner Position gehalten wird. Das Material 90 ist hier eine zu bearbeitende Kontaktlinse. Der Spot 16 kann auch durch Verschieben der Fixierungsvorrichtung 32 in Richtung XY' bzw. Z' auf bzw. in dem Material ausgerichtet werden.

Durch die Bearbeitungsvorrichtung 1 wird der von der Strahlquelle 11 erzeugte Laserstrahl 15 auf das Material 90 fokussiert. Ein Fokusdurchmesser von wenigen Mikrometern kann dadurch erreicht werden, dass der Laserstrahl 15 mit einem Strahldurchmesser von einigen Millimetern durch eine Optik mit einigen Zentimetern Brennweite fokussiert wird. Beispielsweise ergibt sich für ein gaußförmiges Strahlprofil ein Fokusdurchmesser von drei Mikrometern, wenn ein Laserstrahl der Wellenlänge 1000 nm und einem Strahldurchmesser von 10 mm mit einer Brennweite von 50 mm fokussiert wird.

Im Allgemeinen besitzt der Laserstrahl 15 am Ausgang der Strahlquelle 11 einen geringeren Strahldurchmesser als zur optimalen Fokussierung notwendig ist. Mit einer Strahlaufweitungsoptik 21 kann der Strahldurchmesser den Erfordernissen angepaßt werden. Bevorzugt kann als Strahlaufweitungsoptik 21 ein auf unendlich eingestelltes Teleskop nach Galilei (Zerstreuungslinse plus Sammellinse) eingesetzt werden. Hierbei entsteht kein Zwischenfokus, der unter Umständen schon zu einem optischen Durchbruch in Luft führen könnte. Damit ist die verbleibende Laserenergie höher und das Strahlprofil gleichbleibend gut. Bevorzugt ist die Verwendung von Linsensystemen, die zu optimalen Abbildungseigenschaften des Teleskops führen. Durch Justage des Teleskops können auch Fertigungsschwankungen in der Strahldivergenz der Strahlquelle 11 ausgeglichen werden.

In diesem Ausführungsbeispiel wird der Laserfokus scannend über bzw. durch das Material bewegt. Der Laserfokus bzw. Laserspot 16 wird so dreidimensional mit Mikrometergenauigkeit gescannt. Der aufgeweitete Laserstrahl 15' wird senkrecht zur ursprünglichen Strahlrichtung durch eine Ablenkeinrichtung 23 abgelenkt. Hierbei verschiebt sich die Lage des Fokus 16 nach der Fokussieroptik 24 senkrecht zur ursprünglichen Strahlrichtung. Damit kann der Fokus in einer Fläche, die im Wesentlichen eben und senkrecht zur Laserstrahlrichtung ist (X/Y-Richtung) bewegt werden. Die Bewegung parallel zur Strahlrichtung (Z-Richtung) kann zum einen durch bewegen des Werkstücks erfolgen (siehe Pfeil Z'). Die Scan-Algorithmen sind dann bevorzugt so ausgelegt, dass das Werkstück nur langsam bewegt werden muß und die schnellen Scannbewegungen von der Ablenkeinheit ausgeführt werden. Zum anderen kann auch die Fokussieroptik parallel zur Laserstrahlrichtung bewegt werden (Pfeil Z), um damit den Fokus in Z-Richtung zu senken. Insbesondere bei medizinischen Applikationen ist die zweite Methode bevorzugt, da der Patient im allgemeinen nicht schnell genug bewegt werden kann.

Das bearbeitete Material 90 wird relativ zum Lasergerät in einer Fixier- und Justagevorrichtung 32 fixiert. Bevorzugt wird hier die Fixiervorrichtung senkrecht und parallel zur Strahlrichtung justiert, um das Schnittmuster an die vorgesehene Stelle im Material 90 plazieren zu können. Ein mit dem bearbeitenden Laserstrahl 15, 15' kolinearer sichtbarer Laserstrahl aus einem Pilotlaser 27 unterstützt hierbei die Justierung.

Zur Strahlführung und zur Feinjustage der Strahllage zwischen den einzelnen Komponenten sind Spiegel bzw. Spiegelpaare 22 vorgesehen. Die Beschaffenheit der Spiegel wird bevorzugt so gewählt, dass der bearbeitende Laserstrahl diesen nicht zerstört, die Spiegel hoch reflektierend für die Wellenlänge des Bearbeitungslasers und hinreichend reflektierend für den Pilotlaser sind. Bevorzugt wird die Beschichtung so gewählt, dass der Spiegel die Laserpulsdauer nicht wesentlich verlängert. Besonders bevorzugt wird mindestens einer der Spiegel ein sogenannter "Chirped Mirror" sein, mit dem die Dispersion aller im Strahlengang vorhandenen Optiken kompensiert werden kann, um optimal kurze Pulse im Bearbeitungsfokus zu erzielen.

In Figur 2 ist ein weiteres Ausführungsbeispiel der vorliegenden Laserbearbeitungsvorrichtung mit Operationsmikroskop gezeigt. Der Aufbau entspricht im Wesentlichen dem Aufbau in Figur 1. Gleiche Teile sind mit gleichen Bezugszeichen gekennzeichnet. Als Material 90 ist hier ein menschliches Auge vorgesehen. Es soll nun beispielhaft dieses Lasergerät detailiert beschrieben werden, mit dem präzise Schnitte in der Hornhaut des menschlichen Auges eingebracht werden können. Dabei soll eine kreisförmige Fläche, die der Krümmung der Hornhaut folgt und zur optischen Achse des Auges zentriert ist, mit fs-Laserpulsen innerhalb der Hornhaut geschnitten werden. Durch einen kreissegmentförmigen Randschnitt von der Kreisfläche bis zur Außenseite der Hornhaut entsteht ein Hornhautlappen (Flap), der nach dem Laserschnitt zur Seite geklappt werden kann.

Solche ein Flap dient zur Vorbereitung einer LASIK-Operation, bei der durch Laserabtrag die Dicke der Hornhaut so variiert wird, dass refraktive Fehler des Auges kompensiert werden. Bisher wird dieser Schnitt mit einem mechanischen Keratom durchgeführt, was ein hohes Maß an Übung beim Arzt voraussetzt und risikobehaftet ist. Zusätzlich kann durch eine weitere gekrümmte Kreisfläche, die zusammen mit der ersten Kreisfläche des Flaps ein Lentikel umschließt, das nach Aufklappen des Flaps entnommen werden kann, im gleichen Arbeitsgang eine refraktive Korrektur der Hornhaut erfolgen.

Bei der besonderen Ausgestaltung der Erfindung wird das Auge durch einen Saugring 32 an ein Kontaktglas 31 gedrückt, das entweder eben ist, oder bevorzugt der Krümmung der Hornhaut im Wesentlichen angepaßt ist. Der Saugring ist fest mit dem Austrittsfenster des Lasergerätes verbunden, was für eine definierte Lage der Hornhaut relativ zum Laserfokus sorgt. Der aufgeweitete Femtosekunden-Laserstrahl wird mit einer Optik 24 in die Hornhaut fokussiert. Ein Strahlteiler, der für die Laserwellenlänge hochreflektierend und für sichtbares Licht transmitierend ist, spiegelt den Laserstrahl in den Strahlengang eines Operationsmikroskopes ein, das zur Beobachtung und Zentrierung des Auges dient. Die Fokussieroptik 24 bildet dabei einen Teil des Mikroskopobjektives. Zusammen mit einer bündelnden Optik kann ein reelles Zwischenbild der Hornhaut erzeugt werden, das man sich mit dem Stereo-Okular 80 räumlich anschauen kann. Die Strahlablenkeinheit 23 lenkt den aufgeweiteten Laserstrahl 15 senkrecht zu dessen Ausbreitungsrichtung aus. Somit kann der Laserfokus auf unterschiedliche Punkte in der Hornhaut gerichtet werden. Die Fokustiefe kann durch verschieben der Fokussieroptik 24 längs der optischen Achse oder durch Anpassung der Brennweite der Fokussieroptik variiert werden.

Vorzugsweise werden mit der Ablenkeinheit Kreisbahnen abgefahren. Zum Schneiden der Kreisfläche wird der Kreisradius von Kreisbahn zu Kreisbahn verringert und die Repetitionsrate so angepasst, dass ein einheitlicher SpotAbstand beibehalten wird. Die Fokustiefe wird von Kreisbahn zu Kreisbahn so angepasst, dass der Schnitt der Krümmung der Hornhaut folgt. Sollen astigmatische Korrekturen der Sehkraft (Zylinderkorrektur) eingebracht werden, kann die Fokustiefe während der Kreisbahn zweimal auf und ab bewegt werden, so das ein Lentikel mit Zylinderlinsenanteil entsteht. Für die Flapkante wird bei festem Radius die Fokustiefe vom Flapboden langsam bis zur Außenseite der Hornhaut verschoben, so dass ein Zylindermantel entsteht. Auf einem Bogenstück der dabei beschriebenen Kreise muss der Laserstrahl unterbrochen werden, um einen "Hing", an dem der präparierte Flap festgehalten wird, zu belassen. Dazu wird einfach das Auskoppeln von Laserpulsen aus der Strahlquelle 11 unterbrochen.

Die Strahlquelle 11 ist ein "cavity-dumped" Femtosekunden-Oszillator, der bevorzugt direkt diodengepumpt und damit einfach und zuverlässig ist. Der emittierte Laserstrahl 15 wird bevorzugt mit einem Galilei-Teleskop auf 1-2 cm Strahldurchmesser aufgeweitet. Kolinear zum aufgeweiteten Laserstrahl 15 wird ein sichtbarer Laserstrahl aus einem Pilotlaser 27 überlagert, der dann zusammen mit dem Bearbeitungslaserstrahl gescannt und fokussiert wird. Der Strahlteiler 57 ist für diesen Zweck transparent für die Femtosekunden-Laserwellenlänge und reflektierend für den Pilotstrahl.

Die Vielfalt der möglichen Schnittfiguren hängt nur von den Scannalgorithmen ab. Prinzipiell ist ein Lasergerät wie beschrieben zu einer Vielzahl von Applikationen (beispielsweise für refraktive Korrekturen der Sehkraft), bei denen Schnitte oder Strukturumwandlungen innerhalb der transparenten Bestandteile des Auges (Hornhaut, Linse, Glaskörper) vorgenommen werden sollen, geeignet. Damit übertrifft die Erfindung selbst in diesem kleinen Teilbereich der Anwendung in Universalität und Präzision (Schonung von umliegendem Gewebe) bestehende Technologien bei weitem.

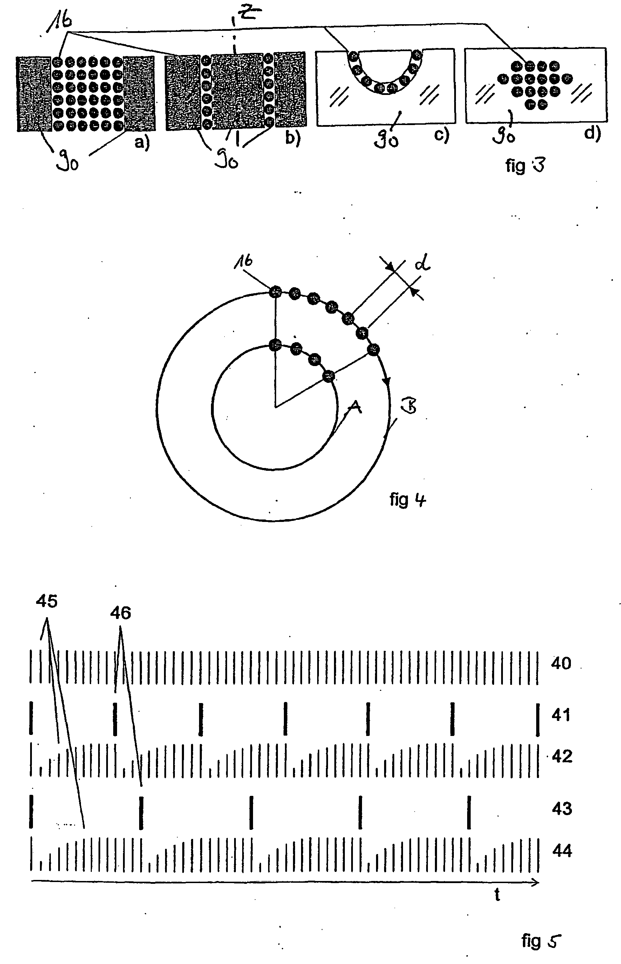

In **Figur 3** sind in den Unterdarstellungen 3 a bis d Anwendungsbeispiele von Schnittgeometrien gezeigt, die mit dem erfindungsgemäßen Lasersystem realisiert werden können. Diese Anwendungen sind nur beispielhaft - es können beliebige weitere Geometrien realisiert werden. Im Fokus 16 des Lasers wird der Zusammenhalt des Materials 90 aufgehoben (Photodisruption). Im Allgemeinen geht das mit einer lokalen Verdampfung des Materials einher. Nach der Einwirkung des Laserpulses ist in einem kleinen Volumen, der Kavitationsblase (im folgenden auch Spot 16 genannt) das Materialgefüge dauerhaft oder für einen bis mindestens zum Ende der Bearbeitungsdauer dauernden Zeitraum aufgehoben. Der Einsatz eines hart fokussierten Femtosekunden-Lasers bzw. - Oszillators bietet damit die präziseste Lokalisierung der Laserwirkung. In dem scharf begrenzten Fokusvolumen wird damit das Materialgefüge zerstört, während es in dicht benachbarten Bereichen (schon weniger als ein Mikrometer entfernt) im Allgemeinen keine Veränderung des Materials stattfindet. Daraus ergibt sich eine hohe Bearbeitungspräzision bei Schonung benachbarter Materialregionen.

Für Schnitte und Strukturierungen werden eine Vielzahl von einzelnen Spots, die das Materialgefüge auflösen, dicht nebeneinander platziert. Der Abstand benachbarter Spots sollte am Ende der Prozedur in der Größenordnung des Spotdurchmessers liegen. In Figur 3 a wird ein vorbestimmtes Volumen (z.B. eine Bohrung im Material) durch vollständiges Ausfüllen des abzutragenden Volumens mit einzelnen Spots 16 generiert. Bei einem solchen nicht transparenten Material geht man dabei schichtweise beginnend mit der dem Laser zugewandten Schicht von Spots vor.

In Figur 3b wird nur der Rand der Bohrung mit Spots überdeckt. Es soll hier ein Schnitt durch das Material gezeigt sein. Die Spots 16 sollen rotationssymmetrisch um die gestrichelt eingezeichnete Achse Z angeordnet sein. Auf diese Weise wird ein Bohrkern in der Mitte des bearbeiteten Materials 90 erzeugt. Der Bohrkern kann anschließend als zusammenhängendes Stück entnommen werden. Die benötigte Anzahl von Laserpulsen verringert sich damit insbesondere bei großen Querschnittsflächen der Bohrung erheblich im Vergleich zur Figur 3 a.

In Figur 3c ist eine Unterschneidung in einem transparenten Material 90 gezeigt. Da die Strahlung vom Material 90 nicht absorbiert wird, sind zusammenhängende Materialstücke durch Plazierung von Spots auf der Schnittkante aus dem Material herauslösbar, wenn dieses an die Oberfläche grenzt.

In Figur 3d ist gezeigt, wie in einem transparenten Material je nach Beschaffenheit des Materials Hohlräume bzw. Strukturierungen (z.B. Änderungen der optischen Eigenschaften) erzeugt werden können.

Für makroskopische Schnittfiguren (im Zentimeterbereich) werden einige Millionen Laserspots benötigt, selbst um nur die Schnittfläche (wie in Figuren 3b und c) dicht genug mit Spots zu überdecken. Für viele Anwendungen (insbesondere medizinische Applikationen) ist es vorteilhaft, die Bearbeitungs- bzw. Behandlungszeit so gering wie möglich zu halten. Die Strahlquelle des Lasergeräts sollte daher in der Lage sein, Laserpulse mit einer hohen Repetitionsrate abzugeben. Kontinuierlich (im cw-modelocking-Betrieb) arbeitende Femtosekunden-Laseroszillatoren nach dem Stand der Technik weisen meistens Repetitionsraten von einigen zehn MHz auf - die Pulsenergien im nJ-Bereich reichen bei hinreichenden großen Brennweiten der Fokussieroptik (Arbeitsabstand von einigen cm) nicht aus, um einen optischen Durchbruch im Material zu erzeugen. Dies wird mit einem erfindungsgemäßen Lasersystem jedoch erreicht.

In Figur 4 wird schematisch ein Ausschnitt aus einem möglichen Scanmuster gezeigt, bei dem die einzelnen von einzelnen Laserpulsen bearbeiteten Spots 16 entlang von Bahnen angeordnet sind, die vom Scanner kontinuierlich abgefahren werden können. Um bei hohen Repetitionsraten der Strahlquelle 11 einen hinreichenden großen Spotabstand zu erzielen, wird der Fokus in mindestens einer von drei Scandimensionen sehr schnell bewegt. Die Scan-Algorithmen werden daher bevorzugt so ausgelegt, dass die Spots entlang von Bahnen, die den natürlichen Bewegungen der Ablenkeinheit entsprechen, platziert werden. Die Bewegung in den anderen zwei Dimensionen kann dann relativ langsam erfolgen. Die natürlichen Bahnen der Ablenkeinheit können z.B. Kreisbahnen sein, die die Ablenkeinheiten mit festen Umlauffrequenzen abfahren kann. Das kann z.B. durch rotierende optische Elemente in der Ablenkeinheit erfolgen. Der Radius der Kreisbahn und die Fokustiefe (Z-Richtung) sind dann die langsam veränderbaren Scangrößen. Diese Variante eignet sich besonders, wenn rotationssymmetrische Schnittfiguren erzeugt werden müssen. Die Repetitionsrate des Lasers läßt sich dann besonders effektiv nutzen, wenn die Umlauffrequenz der Kreisbahnen so gewählt wird, dass bei den größten abzufahrenden Kreisbahnen (B) die volle Repetitionsrate der Strahlquelle zum gewünschten Spotabstand b führt. Werden die Kreisbahnen beim Abfahren des Schnittmusters kleiner im Radius (A), kann die Repetitionsrate der Quelle entsprechend verringert werden, so dass sich wieder der optimale Spotabstand ergibt. Diese Anpassung der Repetitionsrate ist bei einem geeigneten cavity-dumped Oszillator ohne Weiteres möglich. Eine Anpassung der Umlauffrequenz an die Repetitionsrate der Quelle kann technologisch schwieriger sein, insbesondere wenn diese kontinuierlich für jede Kreisbahn (A, B) erfolgt. Für eine Verringerung der Bearbeitungszeit kann aber eine Anpassung der Umlauffrequenz in wenigen Schritten an die kleineren Kreisbahnen von Vorteil sein.

In Figur 5 sind mögliche Folgen von Laserpulsen im und außerhalb des Laserresonators dargestellt. Die Umlauffrequenz der Laserpulse im Oszillator 40 hängt nur von der Resonatorlänge ab und ist für eine bestimmte Strahlquelle vorgegeben und liegt bei Resonatorlängen von wenigen Metern um 100 MHz. Das Prinzip des cavity dumping ist es, einen Teil des im Resonator umlaufenden Pulses 45 zu einem bestimmten Zeitpunkt mit einem schaltbaren optischen Element auszukoppeln. Dabei verlässt ein Großteil der Intracavity-Pulsenergie die Strahlquelle und wird als Ausgangslaserpuls 46 in das Strahlablenksystem geleitet.

Die im Resonator verbleibende Pulsenergie wird nach einer Anzahl von Umläufen im Resonator wieder auf ihren Sättigungswert verstärkt 42 und kann anschließend von neuem ausgekoppelt werden. Die ausgekoppelten Laserpulse 46 haben somit eine geringere Repetitionsrate als die Intracavity-Pulse 45. Die maximale Repetitionsrate ist durch die Zeit bis zum Wiedererlangen der Sättigungspulsenergie nach Auskoppeln eines Laserpulses vorgegeben. Der Verlauf der Intracavity-Pulsenergie entspricht dann in etwa dem in Figur 5 als 42 gezeigten Verhalten.

Die Repetitionsrate kann einfach dadurch verringert werden, dass erst eine gewisse Zeit nach Erreichen der Sättigungspulsenergie erneut ausgekoppelt wird 43, 44. Die Zeit zwischen zwei ausgekoppelten Laserpulsen (Reziproke der Repetitionsrate) kann also in Schritten der Resonatorumlaufzeit frei zwischen unendlich (Repetitionsrate null) und einem Minimum (maximale Repetitionsrate) eingestellt werden. Damit kann die Pulsfolgefrequenz der Ablenkgeschwindigkeit des Strahlablenksystems angepasst werden. Bei typischen Verhältnissen der ausgekoppelten Repetitionsrate zur Umlauffrequenz im Resonator von 1:100 - 1:1000 kann somit die Zahl der pro Umlauf der Ablenkeinheit platzierten Laserspots in minimalen Schritten von 0,1 - 1 % variiert werden. Die Spotabstände auf unterschiedlichen Kreisbahnen streuen dann nur um Bruchteile von einem Prozent.

Mit der vorliegenden Erfindung wurde ein Lasergerät bereitgestellt, das eine Femtosekunden-Strahlquelle umfaßt und bevorzugt auch Mittel zur Strahlformung und -führung, eine programmierbare Strahlablenkung, eine Strahlfokussierung und einer Vorrichtung zur Positionierung und Fixierung des zu bearbeitenden Materials bzw. Gewebes. Die Vorrichtung ist hinsichtlich Laserpulsparametern, Repetitionsrate und Ablenkgeschwindigkeit und -genauigkeit bevorzugt zum präzisen Schneiden und Strukturieren geeignet. Dabei kann bei für die Laserwellenlänge transparenten Materialien die Bearbeitung auch innerhalb des Materials erfolgen. Das zu entfernende Material muss dabei nicht vollständig vom Laser abgetragen werden, sondern durch geeignete Schnittführung können zusammenhängende Materialregionen intakt entfernt werden. Die Schnittgenauigkeit hängt vom Durchmesser des Laserfokus ab und liegt im Mikrometer- und Submikrometerbereich. Die Verwendung von Laserimpulsen mit einer Pulsdauer deutlich unter einer Pikosekunde reduziert die kolaterale Material- bzw. Gewebeschädigung nahezu vollständig und trägt damit wesentlich zur Bearbeitungspräzision bei und erlaubt insbesondere die medizinische Behandlung von sensiblen Geweben in-vivo.

### Bezugszeichenliste

- 1: Bearbeitungsvorrichtung
- 10: Lasersystem
- 11: Cavity-dumped fs-Oszillator
- 15: Laserstrahl
- 15': aufgeweiteter Laserstrahl
- 16: Laserspot/ Laserfokus
- 20: Strahleinrichtung
- 21: Strahlformungseinrichtung/ Strahlaufweitungsoptik
- 22: Strahlführungseinrichtung/ Spiegel
- 23: Strahlablenkungseinrichtung/ Scanner
- 24: Strahlfokussierungseinrichtung/ Linsen
- 27: Pilotlaser
- 30: Haltevorrichtung
- 31: Positionierungsvorrichtung/ Kontaktglas
- 32: Fixierungsvorrichtung/ Saugring
- 80: Beobachtungsmikroskop
- 90: Material/ Auge

## Patentansprüche

1. Vorrichtung (1) zur präzisen Bearbeitung von Material (90), insbesondere organischem Material, umfassend
ein gepulstes Lasersystem (10) mit
einer Strahlquelle (15),
wobei ein cavity-dumped fs-Oszillator (11) als Strahlquelle vorgesehen ist,
**dadurch gekennzeichnet, dass**
Haltevorrichtungen (30) zur Positionierung (31) und/oder Fixierung (32) des zu bearbeitenden Materials (90) vorgesehen sind, und
die Vorrichtung (1) eingerichtet ist Schnitte oder Strukturumwandlungen innerhalb von transparenten Bestandteilen eines Auges durchzuführen.

2. Vorrichtung (1) nach Anspruch 1, umfassend
eine Strahleinrichtung mit
mindestens einem Mittel zur Strahlablenkung eines Arbeitsstrahls der Strahlquelle (15), wobei der Arbeitsstrahl auf das Material (90) applizierbar ist.

3. Vorrichtung (1) nach Anspruch 2, wobei
das Mittel zur Strahlablenkung ein Mittel zur Freigabe von Laserpulsen umfasst,
eine Pulsaussendung mit der Strahlablenkung korreliert und wobei
die Pulsaussendung in Abhängigkeit der Strahlablenkung angesteuert wird.

4. Vorrichtung (1) nach einem der Ansprüche 1 bis 3, wobei
durch das gepulste Lasersystem (10) ein Laserfokus erzeugbar ist,
der dreidimensional durch das Material scannbar ist.

5. Vorrichtung (1) nach Anspruch 4, wobei
der Laserfokus auf in einer Dimension periodisch wiederkehrenden Bahnen ablenkbar ist.

6. Vorrichtung nach Anspruch 1, **dadurch gekennzeichnet, dass**
weiterhin Strahleinrichtungen (20) zur Strahlformung (21) und/ oder Strahlführung (22) und/oder Strahlablenkung (23) und/oder Strahlfokussierung (24) vorgesehen sind.

7. Vorrichtung nach Anspruch 2, **dadurch gekennzeichnet, dass**
die Strahleinrichtungen (20) programmierbar sind.

8. Vorrichtung nach einem der vorhergehenden Ansprüche, **dadurch gekennzeichnet, dass**
durch den cavity-dumped fs-Oszillator (11) Laserpulse von 100 nJ bis 100 µJ, bevorzugt 1 µJ Pulsenergie bereitstellbar sind und/oder
mit einer Pulsdauer von 100 fs bis 1 ps, bevorzugt mit einer Pulsdauer von 200 fs bis 300 fs bereitstellbar sind.

9. Vorrichtung nach einem der vorhergehenden Ansprüche, **dadurch gekennzeichnet, dass**
durch den cavity-dumped fs-Oszillator (11) Laserpulse mit Repetitionsraten von 10 kHz bis 10 MHz bereitstellbar sind.

10. Vorrichtung nach einem der vorhergehenden Ansprüche, **dadurch gekennzeichnet, dass**
durch die Strahleinrichtungen (20) ein Arbeitsstrahl der Strahlquelle in geometrisch vorbestimmbaren Formen in zeitlich vorbestimmbarem Verlauf auf das Material (90) applizierbar ist.

11. Vorrichtung nach Anspruch 1, **dadurch gekennzeichnet, dass**
der gepulste Arbeitsstrahl durch die Strahlablenkungseinrichtung (23) auf das Material applizierbar ist und währenddessen die Repetitionsrate modifizierbar ist.

## Claims

1. Device (1) for precise processing of material (90), in particular organic material, comprising
a pulsed laser system (10) with
a beam source (15),
wherein a cavity-dumped fs oscillator (11) is provided as beam source,
**characterized in that**
holding devices (30) are provided for positioning (31) and/or fixing (32) the material (90) to be processed, and
the device (1) is set up to carry out incisions or structural changes within transparent parts of an eye.

2. Device (1) according to claim 1, comprising
a beam apparatus with
at least one means for deflecting a working beam of the beam source (15), wherein
the working beam can be applied to the material (90).

3. Device (1) according to claim 2, wherein
the means for beam deflection comprises a means for releasing laser pulses,
a pulse emission correlates with the beam deflection and wherein the pulse emission is triggered depending on the beam deflection.

4. Device (1) according to one of claims 1 to 3, wherein
a laser focus, which can be scanned three-dimensionally through the material,
can be produced by the pulsed laser system (10).

5. Device (1) according to claim 4, wherein
the laser focus can be deflected onto paths which recur periodically in one dimension.

6. Device according to claim 1, **characterized in that**
furthermore, beam apparatuses (20) are provided for beam formation (21) and/or beam guidance (22) and/or beam deflection (23) and/or beam focussing (24).

7. Device according to claim 2, **characterized in that**
the beam apparatuses (20) can be programmed.

8. Device according to one of the previous claims, **characterized in that**
the cavity-dumped fs oscillator (11) can provide laser pulses with a pulse energy of 100 nJ to 100 µJ, preferably 1 µJ and/or
with a pulse duration of 100 fs to 1 ps, preferably with a pulse duration of 200 fs to 300 fs.

9. Device according to one of the previous claims, **characterized in that**
laser pulses with repetition rates of 10 kHz to 10 MHz can be provided by the cavity-dumped fs oscillator (11).

10. Device according to one of the previous claims, **characterized in that**
a working beam of the beam source can be applied to the material (90) in geometrically predeterminable shapes and in a chronologically predeterminable course by the beam apparatuses (20).

11. Device according to claim 1, **characterized in that**
the pulsed working beam can be applied to the material by the beam deflection apparatus (23) and in the meantime the repetition rate can be modified.

## Revendications

1. Dispositif (1) pour le traitement précis d'un matériau (90), notamment d'un matériau organique, comprenant :
un système laser pulsé (10) avec une source de rayonnement (15) ;
un oscillateur fs à vidage de cavité (11) servant de source de rayonnement étant prévu ;
**caractérisé en ce que** :
des dispositifs de maintien (30) sont prévus pour le positionnement (31) et/ou la fixation (32) du matériau (90) à traiter ; et
le dispositif (1) est conçu pour réaliser des coupes ou des transformations structurelles à l'intérieur des composants transparents d'un oeil.

2. Dispositif (1) selon la revendication 1, comprenant un dispositif de rayonnement avec au moins un moyen de déviation de faisceau à partir d'un faisceau de travail de la source de rayonnement (15), le faisceau de travail pouvant être appliqué sur le matériau (90).

3. Dispositif (1) selon la revendication 2, le moyen de déviation de faisceau comprenant un moyen de déclenchement d'impulsions laser, une émission d'impulsions corrélée à la déviation de faisceau et l'émission d'impulsions étant commandée en fonction de la déviation de faisceau.

4. Dispositif (1) selon l'une quelconque des revendications 1 à 3, le système laser pulsé (10) permettant de produire un foyer laser pouvant être balayé de façon tridimensionnelle par le matériau.

5. Dispositif (1) selon la revendication 4, le foyer laser pouvant être dévié sur des couloirs revenant périodiquement dans une dimension.

6. Dispositif selon la revendication 1, **caractérisé en ce que** des dispositifs de rayonnement (20) supplémentaires sont prévus pour la formation de faisceau (21) et/ou le guidage de faisceau et/ou la déviation de faisceau (23) et/ou la focalisation de faisceau (24).

7. Dispositif selon la revendication 2, **caractérisé en ce que** les dispositifs de rayonnement (20) sont programmables.

8. Dispositif selon l'une quelconque des revendications précédentes, **caractérisé en ce que** l'oscillateur fs à vidage de cavité (11) permet de mettre à disposition des impulsions laser de 100 nJ à 100 µJ, de façon préférée une énergie d'impulsion de 1 µJ et/ou avec une durée d'impulsion de 100 fs à 1 ps, de façon préférée avec une durée d'impulsion de 200 fs à 300 fs.

9. Dispositif selon l'une quelconque des revendications précédentes, **caractérisé en ce que** l'oscillateur fs à vidage de cavité (11) permet de mettre à disposition des impulsions laser avec des vitesses de répétition de 10 kHz à 10 MHz.

10. Dispositif selon l'une quelconque des revendications précédentes, **caractérisé en ce que** les dispositifs de rayonnement (20) permettent d'appliquer un faisceau de travail de la source de rayonnement sur le matériau (90) dans des formes géométriquement prédéterminables, dans des courbes prédéterminables de temps.

11. Dispositif selon la revendication 1, **caractérisé en ce que** le faisceau de travail pulsé peut être appliqué sur le matériau par le biais du dispositif de déviation de faisceau (23), permettant ainsi de modifier la vitesse de répétition.
